# EUROPEAN PATENT SPECIFICATION

(11) **EP 4 304 563 B1**
(45) Date of publication and mention of the grant of the patent: **04.12.2024**
(21) Application number: 22700765.5
(22) Date of filing: 12.01.2022
(51) Int. Cl.: A61K 9/48

(54) **PULLULAN CAPSULES**
PULLULANKAPSELN
CAPSULES DE PULLULANE

(30) Priority: 12.03.2021 EP 21162354
(43) Date of publication of application: 17.01.2024
(73) Proprietor: Capsugel Belgium NV, 2880 Bornem (BE)
(72) Inventor: TARDY, Claire, 68000 Colmar (FR); VANQUICKENBORNE, Stefaan, 2880 Bornem (BE)
(74) Representative: Weber, Joachim
(86) International application number: PCT/EP2022/050500
(87) International publication number: WO 2022/189045

(56) References cited:
- CN-A- 106 109 437
- CN-A- 106 236 730

## Description

The invention discloses a hard capsule shell comprising pullulan, low acyl gellan, high acyl gellan and KCl.

### BACKGROUND OF THE INVENTION

Hard capsules are used as dosage forms for pharmaceuticals or nutraceuticals.

US 3,258,115 discloses a two-piece hard gelatin capsule, the gelatin forms the shell of the capsule.

Further hard capsule shell compositions are disclosed in CN 106 109 437 B and CN 106 236 730 B.

Gelatin is commonly derived from collagen taken from animal body parts which is not acceptable for vegetarians.

There as a need for a hard capsule which does not contain gelatin in its shell but instead a polymer from non-animal source.

This need was met by a hard capsule made of pullulan.

### Abbreviations and definitions used in this specification

- API: active pharmaceutical ingredient
- CAP: cellulose acetate phthalate
- HAG: High acyl gellan, CAS 71010-52-1, also called native gellan with two acyl substituents acetate and glycerate
- HPMC: hydroxypropyl methylcellulose, also called hypromellose or Cellulose, 2-hydroxypropyl methyl ether or cellulose hydroxypropyl methyl ether, CAS 9004-65-3
- HPMCAS: hydroxypropyl methylcellulose acetate succinate, hypromellose acetate succinate
- HPMCP: hydroxypropyl methylcellulose phthalate
- LAG: Low acyl gellan , CAS 71010-52-1, prepared from High acyl gellan by removing the acyl groups.
- LOD: loss on drying
- pullulan: CAS 9057-02-7
- PVA: polyvinyl alcohol
- SE: Sucrose Ester: Sucrose Monolaurate, CAS 25339-99-5
- SML: sorbitan mono laurate, CAS 1338-39-2

### SUMMARY OF THE INVENTION

Subject of the invention is a hard capsule shell, CAPSSHELL, comprising
95 to 98.7 wt% of pullulan;
0.2 to 0.5 wt% of low acyl gellan;
0.1 to 0.5 wt% of high acyl gellan;
1 to 2 wt% of KCl;
with the wt% being based on the dry capsule weight.

### DETAILED DESCRIPTION OF THE INVENTION

CAPSSHELL is a two piece hard capsule shell. The two pieces are the cap and the body of the capsule shell which are telescopically joined with each other when closing CAPSSHELL.

Preferably, CAPSSHELL does not comprise gelatin, HPMC, PVA or modified starch. Preferably, CAPSSHELL does not comprise a polymer selected from the group of HPMCAS, HPMCP, CAP, and polyacrylic acid copolymers.

More preferably, CAPSSHELL does not comprise any other film forming polymer besides pullulan.

Low acyl gellan, high acyl gellan and their combination are known to the skilled person as being gelling agents.

KCl, when used in combination with a gelling agent, is known to the skilled person as being a gelling aid. So the combination of low acyl gellan, high acyl gellan and KCl in CAPSSHELL is a combination of a gelling agent and a gelling aid.

Preferably, CAPSSHELL does not contain a gelling agent other than the combination of low acyl gellan and high acyl gellan; and CAPSSHELL does not contain a combination of a gelling agent with a gelling aid other than the combination of low acyl gellan, high acyl gellan and KCl.

Preferably, CAPSSHELL comprises
95 to 98.4 wt% of pullulan;
0.25 to 0.45 wt% of low acyl gellan;
0.15 to 0.45 wt% of high acyl gellan;
1.2 to 1.8 wt% of KCl;
with the wt% being based on the dry capsule weight.

More preferably, CAPSSHELL comprises
95 to 98.3 wt% of pullulan;
0.3 to 0.4 wt% of low acyl gellan;
0.2 to 0.4 wt% of high acyl gellan;
1.2 to 1.6 wt% ofKCl;
with the wt% being based on the dry capsule weight.

In one embodiment, CAPSSHELL further comprises sucrose monolaurate.

Preferably, when CAPSSHELL comprises sucrose monolaurate, then CAPSSHELL comprises from 0.05 to 0.25 wt%, more preferably from 0.1 to 0.2 wt%, of sucrose monolaurate, with the wt% being based on the dry capsule weight.

In one embodiment, CAPSSHELL further comprises sorbitan mono laurate.

Preferably, when CAPSSHELL comprises sorbitan mono laurate, then CAPSSHELL comprises from 0.01 to 0.05 wt%, more preferably from 0.02 to 0.04 wt%, of sorbitan mono laurate, with the wt% being based on the dry capsule weight.

In one embodiment, CAPSSHELL further comprises NaHCO₃.

Preferably, when CAPSSHELL comprises NaHCO₃, then CAPSSHELL comprises from 0.02 to 0.06 wt%, more preferably from 0.03 to 0.05 wt%, of NaHCO₃, with the wt% being based on the dry capsule weight.

CAPSSHELL may comprise water. The water may stem from the production process which may be using an aqueous composition for preparing CAPSSHELL, so the water in CAPSSHELL is typically residual water remaining in CAPSSHELL after drying.

The water may also stem from the humidity of the atmosphere, essentially of the relative humidity of the air, surrounding CAPSSHELL. Typical upper limit of the amount of residual water in CAPSSHELL is 20 wt% or less, preferably 15 wt% or less, more preferably 10 wt% or less, the wt% being based on the weight of the CAPSSHELL.

Typical lower limit of the amount of residual water in CAPSSHELL is 1 wt% or more, preferably 2 wt% or more, more preferably 3 wt% or more, the wt% being based on the weight of the CAPSSHELL.

Any of the lower limit of the amount of residual water may be combined with any of the upper limit of the amount of residual water.

For example, the amount of residual water in CAPSSHELL may be from 1 to 20 wt%, preferably from 2 to 15 wt%, more preferably from 3 to 10 wt%, the wt% being based on the weight of the CAPSSHELL.

The amount of residual water may be characterized by the loss on drying, LOD. The dry capsule weight is the capsule weight minus the weight that is lost when the LOD is determined, that is the remaining capsule weight after the LOD determination.

In one embodiment, CAPSSHELL consists of pullulan, low acyl gellan, high acyl gellan, KCl, sucrose monolaurate, sorbitan mono laurate, NaHCO₃ and water;
preferably, CAPSSHELL contains at least 95 wt% of pullulan, with the wt% being based on the weight of the CAPSSHELL.

Further subject of the invention is a method for preparation of CAPSSHEL, wherein CAPSSHELL is prepared by dip molding;
with CAPSSHELL as defined herein, also with all its embodiments.

In dip molding an aqueous mixture of the components of CAPSSHELL, this aqueous mixture is called melt.

A mold pin is dipped into the melt and then extracted from the melt. The melt on the outer surface of the mold pin solidifies to form a film, which is still a wet gel. Then the film is dried at 20 to 80 °C. After drying, unnecessary portions thereof are cut off to produce a body and a cap respectively, for producing a body a respectively shaped mold pin is used for the dip molding, and for producing the cap a respectively shaped mold pin is used for the dip molding. A pair of the body and the cap constitutes CAPSSHELL; by telescopically engaging the cap with the body a closed CAPSSHELL is obtained.

Further subject of the invention is CAPSSHELL filled with an active agent, AA, with AA being selected from the group drug, medicament, pharmaceutical, therapeutic agent, nutraceutical, and active pharmaceutical ingredient, API;
with CAPSSHELL as defined herein, also with all its embodiments.

### EXAMPLES

### Definitions, Materials, Abbreviations and Methods used in this specification

- DM: demineralized
- HAG: High acyl gellan, CAS 71010-52-1, KELCOGEL^{®} CG -HA Gellan Gum, from CPKelco, Atlanta, USA
- Indigocarmin: CAS-Nummer 860-22-0, Dye content 85 %, SigmaAldrich
- KCl: Potassium Chloride, CAS 7440-09-7, VWR Chemicals
- LAG: Low acyl gellan , CAS 71010-52-1, KELCOGEL ^{®} CG-LA Gellan Gum, from CPKelco, Atlanta, USA
- LOD: loss on drying
- NaHCO₃: Sodium bicarbonate, CAS 144-55-8, Sigma Aldrich
- PGC: Plantcaps Gellan Capsules
- PC: Standard Plantcaps Capsules, PLANTCAPS^{®} CAPSULES of Capsugel, now a Lonza company, Lonza Ltd, 3930 Visp, Switzerland
- Pullulan: Pullulan PI-20, CAS 9057-02-7, from Hayashibara Co., Ltd., Japan
- SE: Sucrose Ester: Sucrose Monolaurate (L-1695), CAS 25339-99-5, Mitsubishi Chemical Corporation, Japan
- SML: sorbitan mono laurate, CAS 1338-39-2, Lonza Ltd., Visp, Switzerland
- USP: United States Pharmacopeia

### Tube tester

developed in-house by Capsugel, now Lonza Ltd, Visp, Switzerland:

### Method:

The capsule's fracture and elasticity behavior is measured by its resistance to an impact test with a tube.

### Procedure:

1. Store the capsules for five days before testing in desiccators.
   Storage conditions available are: 10, 23, 33, 45 % RH
   Store 50 capsules for each condition in opened boxes. Closes it thereafter to avoid any moisture exchange with the surrounding atmosphere.
2. Place a capsule horizontally on a flat surface.
3. Put the weight of 100 g in the tube.
4. Place the tube on the capsule and push the latch to release the weight.
5. Test 50 capsules.

Use closed boxes for storing after equilibration with chosen RH, and do not take out every 50 capsules at the same time (to avoid any humidity retaking).

Record the number of body, cap or capsule (body and cap) broken.

### Media preparation

### pH 1.2 USP:

Dissolve 2 g of sodium chloride in 700 ml of demineralized water.

Add 7 ml of concentrated hydrochloric acid (Merck, 37 % in water) under stirring.

Complete with demineralized water to 1000 ml.

### DM water USP:

from Elix^{®} 20 Water Purification System, Millipore, Merck KGaA, Darmstadt, Germany

### pH 6.8 USP:

Solution A: KH₂PO₄ 0.2 M
Dissolve 27.22 g of potassium dihydrogen phosphate (KH₂PO₄) in 1000 ml of demineralized water
Solution B: NaOH 0.2 M
Dissolve 8.0 g of sodium hydroxide (NaOH) in 1000 ml of demineralized water pH 6.8 USP:
Mix 250 ml of solution A and 112 ml of solution B.
Complete with demineralized water to 1000 ml

### Example 1: Preparation of Plantcaps Gellan Melt

| | **Table 1** | |
|---|---|---|
| **Step 1** | **Preparation of gellan melt** | **[kg]** |
| | LAG | 0.35 |
| | HAG | 0.3 |
| | NaHCO3 | 0.04 |
| | Ion exchanged water | 335.3 |
| | Total weight | 335.99 |
| | | |

| **Step 2** | **Preparation of solution of KCl** | |
|---|---|---|
| | KCl | 1.5 |
| | water | 13.5 |
| | Total weight | 15 |
| | Content: 10 wt% KCl based on weight of solution of KCl | |
| | | |

| **Step 3** | **Preparation of solution of surfactants** | |
|---|---|---|
| **Step 3a** | **Preparation of SE solution** | |
| | SE | 0.14 |
| | Water | 1.26 |
| | Total | 1.4 |
| | Content: 10 wt% SE based on weight of SE solution | |

| **Step 3b** | **Preparation of SML solution** | |
|---|---|---|
| | SML | 0.03 |
| | Water | 0.27 |
| | Total | 0.3 |
| | Content: 10 wt% SML based on weight of SML solution | |
| | | |

| **Step 4** | **Preparation of pullulan melt** | |
|---|---|---|
| | gellan melt, prepared according to Step 1 | 335.99 |
| | Solution of KCl, prepared according to Step 2 | 15 |
| | pullulan | 100 |
| | Total weight | 450.99 |

Preparation of gellan melt in Step 1 was done by filling a vessel with ion exchanged water of 80 to 85 °C into which NaHCO3 was charged and mixed for 5 min. A blend of gellans (LAG and HAG) was then added and mixed for 200 min.

A solution of 10 % KCl, prepared according to Step 2, was added into the vessel which was then cooled to 68 °C.

Then pullulan was charged and the mixture was mixed for 60 min. Step 4 in Table 1 gives a summary.

The mixture was kept stirring at 68 °C for 60 min for debubbling until no bubbles were observed anymore and then for further 90 min for ageing, providing the pullulan melt.

The pullulan melt was transferred to a transfer tank at 65 °C.

1.4 kg of SE solution, prepared according to step 3a, and 0.3 kg of SML solution, prepared according to step 3b, were mixed to provide a surfactant solution

The surfactants solution was added to the pullulan melt in the transfer tank; with gentle mixing and maintaining 65 °C, providing the Plantcaps Gellan Melt for further use for dip molding.

**Table 1 gives the amounts of the components that were used. Table 2 gives the composition of the Plantcaps Gellan Melt and of the final capsule based on dry weight of capsule.**

| **Table 2** | | | |
|---|---|---|---|
| **Plantcaps Gellan Melt** | | | |
| | **Melt** | **wt% based on weight of melt** | **wt% based on dry capsule weight** |
| | **[kg]** | **[wt%]** | **[wt%]** |
| Pullulan | 100 | 22.09 | 97.69 |
| LAG | 0.35 | 0.077 | 0.34 |
| HAG | 0.3 | 0.066 | 0.29 |
| KCl | 1.5 | 0.331 | 1.47 |
| | | | |
| NaHCO3 | 0.04 | 0.009 | 0.04 |
| SE | 0.14 | 0.031 | 0.14 |
| SML | 0.03 | 0.007 | 0.03 |
| Ion exchanged water 335.3 + 13.5 + 1.26 + 0.27 =350.33 | 350.33 | 77.389 | --- |
| **Total weight** | **452.69** | **100.0** | **100.0** |

### Example 2: Preparation of Plantcaps Gellan Capsules, PGC

Plantcaps Gellan Melt, prepared according to example 1, was used to produce shells in form of capsule halves (bodies and caps) with capsule size 2 and standard target weights (capsule weight 61 +/- 4 mg) through conventional dip molding by dipping stainless steel mold pins with a temperature of 22 °C into the Plantcaps Gellan Melt with a temperature of 60 °C. A film was formed on the mold pins. After first drying of the film on the mold pins at 26 °C and 44 % RH for 15 to 20 min, a second drying at 32 °C and 25 to 30 % RH for 30 min was applied and finally a drying at 22 °C and 50 % RH was applied before stripping the capsule parts from the metal pins.

The capsule halves were removed from the mold pins.

Capsules were assembled by joining always one cap with one body.

### Example 3: Testing of capsule - Mechanical Performance

The fracture behavior of the capsules, prepared according to Example 2, was measured by their resistance to an impact test with a tube tester. In preparation of the test the capsules were stored for equilibration for five days under four storage conditions in desiccators: 10, 23, 33 and 45 % RH in order to obtain capsules with different LOD. The number of capsules tested was 50 per each RH value and the number of broken bodies or broken caps was recorded. Percentage of broken capsules is presented in Table 3.

### Capsules:

### PGC Plantcaps Gellan Capsules prepared according to Example 2

| | **Table 3** | |
|---|---|---|
| | **Broken Capsules [%]** | **RH of Storage Condition [%]** |
| **PGC** | 100 | 2.5 |
| | 58 | 10 |
| | 2 | 16 |
| | 0 | 23 |
| | 0 | 33 |
| | 0 | 45 |

The mechanical performance is similar for the PGC and the PC, the deviation is within the usual variation of the tube test.

### Example 4: Testing of capsules - Disintegration of Capsules

Capsules, prepared according to Example 2, were tested for disintegration in a disintegration tester, Sotax DT2, SOTAX AG, 4147 Aesch, Switzerland, in accordance to the USP specifications.

For this test the capsules were filled with 320 +/- 10 mg of a blend of Lactose containing 0.1 wt% of Indigocarmin dye.

The capsules were placed in the different media at 37 °C +/- 1 °C and the time of first leak, of capsule emptying and of total disintegration of the capsule were recorded. 6 capsules per media were tested and the respective average time was calculated.

Results are given in Table 4

### Capsules:

### PGC Plantcaps Gellan Capsules prepared according to Example 2

| **Table 4** | | | | |
|---|---|---|---|---|
| | | **Time [min]** | | |
| | | **pH 1.2 USP** | **DM water USP** | **pH 6.8 USP** |
| **PGC** | **First leak** | 02:51 | 01:53 | 02:05 |
| | **Capsule emptied** | 08:55 | 04:27 | 05:19 |
| | **Total disintegration** | 14:34 | 07:17 | 08:28 |

### Example 5: Testing on capsules - Dissolution of capsules

The capsules, prepared according to Example 2, were tested for dissolution performance in different media: pH 1.2 USP, DM water USP and pH 6.8 USP using a Sotax AT70smart, SOTAX AG, 4147 Aesch, Switzerland, dissolution bath at 37 °C with paddle at 50 rpm, acetaminophen dissolution was followed with Perkin Elmer Lambda 25 UV / VIS spectrometer at 300 nm wavelength. Capsules were filled with Acetaminophen (APAP, Sigma Aldrich Ref A5000).

Table 5 shows the results as measured % of dissolution of the APAP contained in the capsules over time.

### Abbreviations in Table 5:

- PGC: Plantcaps Gellan Capsules prepared according to Example 2
- M: Media:
- 1.2: pH 1.2 USP
- DM: DM water USP
- 6.8: pH 6.8 USP

| | **Table 5** | | | | | | | | | | | | |
|---|---|---|---|---|---|---|---|---|---|---|---|---|---|
| | **time [min]** | 0 | 3 | 6 | 9 | 12 | 15 | 20 | 25 | 30 | 45 | 60 | 75 |
| | **M** | | | | | | | | | | | | |
| **PGC** | **1.2** | 0 | 0.2 | 0.4 | 0.7 | 1.1 | 1.7 | 3.6 | 9.3 | 13.9 | 23.9 | 33.4 | 41.2 |
| | **DM** | 0 | 0.2 | 1.1 | 7.2 | 13.9 | 20.6 | 31.3 | 43.0 | 52.6 | 77.6 | 91.3 | 97.6 |
| | **6.8** | 0 | 0.1 | 0.3 | 0.6 | 0.9 | 1.2 | 1.7 | 4.4 | 6.5 | 22.4 | 32.2 | 43.2 |

## Claims

1. A hard capsule shell, CAPSSHELL, comprising
95 to 98.7 wt% of pullulan;
0.2 to 0.5 wt% of low acyl gellan;
0.1 to 0.5 wt% of high acyl gellan;
1 to 2 wt% of KCl;
with the wt% being based on the dry capsule weight.

2. CAPSSHELL according to claim 1, wherein
CAPSSHELL does not comprise gelatin, HPMC, PVA or modified starch.

3. CAPSSHELL according to claim 1 or 2, wherein
CAPSSHELL does not comprise a polymer selected from the group of HPMCAS, HPMCP, CAP, and polyacrylic acid copolymers.

4. CAPSSHELL according to one or more of claims 1 to 3, wherein
CAPSSHELL does not comprise any other film forming polymer besides pullulan.

5. CAPSSHELL according to one or more of claims 1 to 4, wherein
CAPSSHELL does not contain a gelling agent other than the combination of low acyl gellan and high acyl gellan; and CAPSSHELL does not contain a combination of a gelling agent with a gelling aid other than the combination of low acyl gellan, high acyl gellan and KCl.

6. CAPSSHELL according to one or more of claims 1 to 5, wherein
CAPSSHELL comprises
95 to 98.4 wt% of pullulan;
0.25 to 0.45 wt% of low acyl gellan;
0.15 to 0.45 wt% of high acyl gellan;
1.2 to 1.8 wt% of KCl;
with the wt% being based on the dry capsule weight.

7. CAPSSHELL according to one or more of claims 1 to 6, wherein
CAPS SHELL comprises
95 to 98.3 wt% of pullulan;
0.3 to 0.4 wt% of low acyl gellan;
0.2 to 0.4 wt% of high acyl gellan;
1.2 to 1.6 wt% ofKCl;
with the wt% being based on the dry capsule weight.

8. CAPSSHELL according to one or more of claims 1 to 7, wherein
CAPSSHELL further comprises sucrose monolaurate.

9. CAPSSHELL according to claim 8, wherein
CAPSSHELL comprises from 0.05 to 0.25 wt% of sucrose monolaurate, with the wt% being based on the dry capsule weight.

10. CAPSSHELL according to one or more of claims 1 to 9, wherein
CAPSSHELL further comprises sorbitan mono laurate.

11. CAPSSHELL according to claim 10, wherein
CAPSSHELL comprises from 0.01 to 0.05 wt% of sorbitan mono laurate, with the wt% being based on the dry capsule weight.

12. CAPSSHELL according to one or more of claims 1 to 11, wherein
CAPSSHELL further comprises NaHCO₃.

13. CAPSSHELL according to claim 12, wherein
CAPSSHELL comprises from 0.02 to 0.06 wt% of NaHCO₃, with the wt% being based on the dry capsule weight.

14. CAPSSHELL according to one or more of claims 1 to 13, wherein
CAPSSHELL consists of pullulan, low acyl gellan, high acyl gellan, KCl, sucrose monolaurate, sorbitan mono laurate, NaHCO₃ and water.

15. CAPSSHELL according to claim 14, wherein
CAPSSHELL contains at least 95 wt% of pullulan, with the wt% being based on the weight of the CAPSSHELL.

16. A method for preparation of CAPSSHEL, wherein
CAPSSHELL is prepared by dip molding;
with CAPSSHELL as defined in one or more of claims 1 to 15.

17. CAPSSHELL filled with an active agent, AA, with AA being selected from the group drug, medicament, pharmaceutical, therapeutic agent, nutraceutical, and active pharmaceutical ingredient;
with CAPSSHELL as defined in one or more of claims 1 to 15.

## Patentansprüche

1. Hartkapselhülle, CAPSSHELL, umfassend
95 bis 98,7 Gew.-% Pullulan;
0,2 bis 0,5 Gew.-% acylarmes Gellan;
0,1 bis 0,5 Gew.-% hoch acyliertes Gellan;
1 bis 2 Gew.-% KC1;
wobei die Gew.-% auf dem Trockengewicht der Kapsel basieren.

2. CAPSSHELL nach Anspruch 1, wobei
CAPSSHELL keine Gelatine, HPMC, PVA oder modifizierte Stärke umfasst.

3. CAPSSHELL nach Anspruch 1 oder 2, wobei
CAPSSHELL kein Polymer aus der Gruppe der HPMCAS, HPMCP, CAP und Polyacrylsäurecopolymere umfasst.

4. CAPSSHELL nach einem oder mehreren der Ansprüche 1 bis 3, wobei
CAPSSHELL neben Pullulan kein weiteres filmbildendes Polymer umfasst.

5. CAPSSHELL nach einem oder mehreren der Ansprüche 1 bis 4, wobei
CAPSSHELL kein anderes Geliermittel als die Kombination aus niedrig acyliertem Gellan und hoch acyliertem Gellan enthält; und CAPSSHELL keine Kombination eines Geliermittels mit einem anderen Gelierhilfsmittel als der Kombination aus niedrig acyliertem Gellan, hoch acyliertem Gellan und KC1 enthält.

6. CAPSSHELL nach einem oder mehreren der Ansprüche 1 bis 5, wobei
CAPSSHELL Folgendes umfasst
95 bis 98,4 Gew.-% Pullulan;
0,25 bis 0,45 Gew.-% acylarmes Gellan;
0,15 bis 0,45 Gew.-% hoch acyliertes Gellan;
1,2 bis 1,8 Gew.-% KC1;
wobei die Gew.-% auf dem Trockengewicht der Kapsel basieren.

7. CAPSSHELL nach einem oder mehreren der Ansprüche 1 bis 6, wobei
CAPSSHELL Folgendes umfasst
95 bis 98,3 Gew.-% Pullulan;
0,3 bis 0,4 Gew.-% acylarmes Gellan;
0,2 bis 0,4 Gew.-% hoch acyliertes Gellan;
1,2 bis 1,6 Gew.-% KCl;
wobei die Gew.-% auf dem Trockengewicht der Kapsel basieren.

8. CAPSSHELL nach einem oder mehreren der Ansprüche 1 bis 7, wobei
CAPSSHELL ferner Saccharosemonolaurat umfasst.

9. CAPSSHELL nach Anspruch 8, wobei
CAPSSHELL 0,05 bis 0,25 Gew.-% Saccharosemonolaurat umfasst, wobei die Gew.-% auf dem Trockengewicht der Kapsel basieren.

10. CAPSSHELL nach einem oder mehreren der Ansprüche 1 bis 9, wobei
CAPSSHELL ferner Sorbitanmonolaurat umfasst.

11. CAPSSHELL nach Anspruch 10, wobei
CAPSSHELL 0,01 bis 0,05 Gew.-% Sorbitanmonolaurat umfasst, wobei die Gew.-% auf dem Trockengewicht der Kapsel basieren.

12. CAPSSHELL nach einem oder mehreren der Ansprüche 1 bis 11, wobei
CAPSSHELL ferner NaHCO₃ umfasst.

13. CAPSSHELL nach Anspruch 12, wobei
CAPSSHELL 0,02 bis 0,06 Gew.-% NaHCO₃ umfasst, wobei die Gew.-% auf dem Trockengewicht der Kapsel basieren.

14. CAPSSHELL nach einem oder mehreren der Ansprüche 1 bis 13, wobei
CAPSSHELL aus Pullulan, Gellan mit niedrigem Acylgehalt, Gellan mit hohem Acylgehalt, KC1, Saccharosemonolaurat, Sorbitanmonolaurat, NaHCO₃ und Wasser besteht.

15. CAPSSHELL nach Anspruch 14, wobei
CAPSSHELL mindestens 95 Gew.-% Pullulan enthält, wobei die Gew.-% auf dem Gewicht der CAPSSHELL basieren.

16. Verfahren zur Vorbereitung von CAPSSHEL, wobei CAPSSHELL im Tauchformverfahren vorbereitet wird;
mit CAPSSHELL, wie in einem oder mehreren der Ansprüche 1 bis 15 definiert.

17. CAPSSHELL, gefüllt mit einem aktiven Mittel, AA, wobei AA ausgewählt ist aus der Gruppe Arzneimittel, Medikament, Pharmazeutikum, therapeutischer Wirkstoff, Nutrazeutikum und aktiver pharmazeutischer Wirkstoff; mit CAPSSHELL, wie in einem oder mehreren der Ansprüche 1 bis 15 definiert.

## Revendications

1. Enveloppe de capsule dure, CAPSSHELL, comprenant
95 à 98,7 % en poids de pullulane ;
0,2 à 0,5 % en poids de gellane à faible teneur en acyle ;
0,1 à 0,5 % en poids de gellane à forte teneur en acyle ;
1 à 2 % en poids de KCl ;
le % en poids étant basé sur le poids de la capsule sèche.

2. CAPSSHELL selon la revendication 1, dans laquelle CAPSSHELL ne contient pas de gélatine, d'HPMC, de PVA ou d'amidon modifié.

3. CAPSSHELL selon la revendication 1 ou 2, dans laquelle
CAPSSHELL ne comprend pas de polymère choisi dans le groupe des copolymères HPMCAS, HPMCP, CAP et d'acide polyacrylique.

4. CAPSSHELL selon l'une ou plusieurs des revendications 1 à 3, dans laquelle
CAPSSHELL ne comprend aucun autre polymère filmogène en dehors du pullulane.

5. CAPSSHELL selon l'une ou plusieurs des revendications 1 à 4, dans laquelle
CAPSSHELL ne contient pas d'agent gélifiant autre que la combinaison de gellane à faible teneur en acyle et de gellane à forte teneur en acyle ; et CAPSSHELL ne contient pas de combinaison d'un agent gélifiant avec un auxiliaire de gélification autre que la combinaison de gellane à faible teneur en acyle, de gellane à forte teneur en acyle et de KCl.

6. CAPSSHELL selon l'une ou plusieurs des revendications 1 à 5, dans laquelle
CAPSSHELL comprend
95 à 98,4 % en poids de pullulane ;
0,25 à 0,45 % en poids de gellane à faible teneur en acyle ;
0,15 à 0,45 % en poids de gellane à forte teneur en acyle ;
1,2 à 1,8 % en poids de KCl ;
le % en poids étant basé sur le poids de la capsule sèche.

7. CAPSSHELL selon l'une ou plusieurs des revendications 1 à 6, dans laquelle
CAPSSHELL comprend
95 à 98,3 % en poids de pullulane ;
0,3 à 0,4 % en poids de gellane à faible teneur en acyle ;
0,2 à 0,4 % en poids de gellane à forte teneur en acyle ;
1,2 à 1,6 % en poids de KCl ;
le % en poids étant basé sur le poids de la capsule sèche.

8. CAPSSHELL selon l'une ou plusieurs des revendications 1 à 7, dans laquelle
CAPSSHELL comprend en outre du monolaurate de saccharose.

9. CAPSSHELL selon la revendication 8, dans laquelle CAPSSHELL comprend de 0,05 à 0,25 % en poids de monolaurate de saccharose, le % en poids étant basé sur le poids de la capsule sèche.

10. CAPSSHELL selon l'une ou plusieurs des revendications 1 à 9, dans laquelle
CAPSSHELL comprend en outre du monolaurate de sorbitane.

11. CAPSSHELL selon la revendication 10, dans laquelle CAPSSHELL comprend de 0,01 à 0,05 % en poids de monolaurate de sorbitane, le % en poids étant basé sur le poids de la capsule sèche.

12. CAPSSHELL selon l'une ou plusieurs des revendications 1 à 11, dans laquelle
CAPSSHELL comprend en outre NaHCO₃.

13. CAPSSHELL selon la revendication 12, dans laquelle CAPSSHELL comprend de 0,02 à 0,06 % en poids de NaHCO₃, le % en poids étant basé sur le poids de la capsule sèche.

14. CAPSSHELL selon l'une ou plusieurs des revendications 1 à 13, dans laquelle
CAPSSHELL est constituée de pullulane, de gellane à faible teneur en acyle, de gellane à forte teneur en acyle, de KCl, de monolaurate de saccharose, de monolaurate de sorbitane, de NaHCO₃ et d'eau.

15. CAPSSHELL selon la revendication 14, dans laquelle CAPSSHELL contient au moins 95 % en poids de pullulane, le % en poids étant basé sur le poids de CAPSSHELL.

16. Procédé de préparation de CAPSSHELL, dans lequel CAPSSHELL est préparée par moulage par immersion ;
avec CAPSSHELL tel que définie dans l'une ou plusieurs des revendications 1 à 15.

17. CAPSSHELL remplie d'un agent actif, AA, AA étant choisi dans le groupe de drogue, de médicament, de remède, de produit pharmaceutique, d'agent thérapeutique, de produit nutraceutique et d'ingrédient pharmaceutique actif ;
avec CAPSSHELL tel que définie dans l'une ou plusieurs des revendications 1 à 15.
